(19) Europäisches Patentamt
European Patent Office
Office européen des brevets

(11) EP 1 596 818 B1

(12) EUROPÄISCHE PATENTSCHRIFT

(45) Veröffentlichungstag und Bekanntmachung des Hinweises auf die Patenterteilung:
**03.05.2006 Patentblatt 2006/18**

(21) Anmeldenummer: **03718674.9**

(22) Anmeldetag: **24.02.2003**

(51) Int Cl.:
***A61K 8/49*** *(2006.01)* ***A61Q 19/04*** *(2006.01)*

(86) Internationale Anmeldenummer:
**PCT/EP2003/001874**

(87) Internationale Veröffentlichungsnummer:
**WO 2004/073675 (02.09.2004 Gazette 2004/36)**

(54) **KOSMETISCHE ZUSAMMENSETZUNG MIT WHITENING-EFFEKT, VERFAHREN ZU IHRER HERSTELLUNG UND IHRE VERWENDUNG**

COSMETIC COMPOSITION WITH A WHITENING EFFECT, METHOD FOR THE PRODUCTION AND USE THEREOF

COMPOSITION COSMETIQUE A EFFET ECLAIRCISSANT, PROCEDE DE PRODUCTION ET UTILISATION DE LADITE COMPOSITION

(84) Benannte Vertragsstaaten:
**CH DE LI**

(43) Veröffentlichungstag der Anmeldung:
**23.11.2005 Patentblatt 2005/47**

(73) Patentinhaber: **Riemschneider, Randolph, Dr. Dr. Prof.**
**14052 Berlin (DE)**

(72) Erfinder: **Riemschneider, Randolph, Dr. Dr. Prof.**
**14052 Berlin (DE)**

(74) Vertreter: **Hartmann, Günter et al**
**RUSCHKE HARTMANN MADGWICK & SEIDE**
**Patent- und Rechtsanwälte**
**Pienzenauerstrasse 2**
**81679 München (DE)**

(56) Entgegenhaltungen:
**EP-A- 0 308 919**

- **DATABASE CAPLUS AMERICAN CHEMICAL SOCIETY; 1989, XP002258107 gefunden im STN Database accession no. 1989:121038 & JP 63 022509 A (SANSEI PHARMACEUTICAL CO LTD) 30. Januar 1988 (1988-01-30)**
- **DATABASE CAPLUS AMERICAN CHEMICAL SOCIETY; 2001, XP002258108 gefunden im STN Database accession no. 2001:874340 & JP 2001 335422 A (MASASHINO MENEKI KENKYUSHO) 4. Dezember 2001 (2001-12-04)**
- **DATABASE CAPLUS AMERICAN CHEMICAL SOCIETY; 1995, XP002258109 gefunden im STN Database accession no. 1995:951810 & JP 07 238010 A (KANEBO LTD) 12. September 1995 (1995-09-12)**
- **DATABASE CAPLUS AMERICAN CHEMICAL SOCIETY; 1994, XP002258110 gefunden im STN Database accession no. 1994:14665 & JP 05 229927 A (KAO CORP) 7. September 1993 (1993-09-07)**
- **DATABASE CAPLUS AMERICAN CHEMICAL SOCIETY; 1977, XP002258112 gefunden im STN Database accession no. 1977:584367 & JP 51 086466 A (NIPPON SODA CO LTD) 29. Juli 1976 (1976-07-29)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft kosmetische Zusammensetzungen mit Whitening-Effekt, d.h. solche, die eine die Melaninbildung (Melanogenese) hemmende Wirkung haben. Sie betrifft insbesondere kosmetische Zusammensetzungen mit Whitening-Effekt, die als Wirkstoff mindestens ein Enol-Lacton der nachstehend angegebenen allgemeinen Formel (I) und/oder eines oder mehrere seiner Tautomeren der ebenfalls nachstehend angegebenen Formeln (Ia- Ic) oder ein Salz, insbesondere ein Erdalkali-, Alkali- oder Ammoniumsalz, davon enthalten, Verfahren zu ihrer Herstellung und ihre Verwendung zur Verhinderung der Bildung brauner Hautflecken und/oder zum Abschwächen (Bleichen) bzw. Entfernen von bereits vorhandenen braunen Hautflekken, bei Säugetieren einschließlich Menschen.

Die Melanin-Pigmentierung bei Mensch und Tier hat seit Beginn der aufgezeichneten Geschichte das Interesse und die Aufmerksamkeit der Menschen gefunden. Dieses Interesse ist zum Teil begründet durch die soziale, kosmetische und Schutz-Signifikanz dieser Pigmentierung. Das Interesse ist noch weiter gestiegen, als Untersuchungen über die mögliche Rolle der Melaninbildung bei der Entwicklung des Melanom-Carcinoms durchgeführt wurden, das bis heute den schlechtesten Ruf unter den bösartigen Tumoren hat. Außerdem wurde gefunden, dass Veränderungen der Melanin-Pigmentierung einen sehr empfindlichen Hinweis auf die funktionelle Aktivität der Melanin bildenden Zellen darstellen und die Möglichkeit bieten, die Beziehung zwischen der funktionellen Aktivität und dem Wachstumsvermögen von Tumorzellen zu verfolgen. Dieses Gebiet ist daher zum Gegenstand vieler Arbeiten auf vielen Gebieten geworden, beispielsweise in der Zytologie zur Untersuchung der Melanin-Granulate; in der Genetik, in der Anthropologie und in der Biologie zur Untersuchung der Beziehung zwischen ererbten Anlagen, Umwelt und Ernährung in ihrem Einfluss auf die Entwicklung des Melanins, und in der organischen und biologischen Chemie für die Untersuchung des chemischen Mechanismus, welcher der Melaninbildung zugrunde liegt.

Melanin, ein braunes bis schwarzes Pigment, das bei Menschen und Wirbeltieren in Melanozyten (Zellen unter der Epidermis) und Melanoblasten unter dem Einfluss von Enzymen, wie z.B. Tyrosinase, und bestimmten Hormonen aus Dihydroxyphenylalanin (Dopa) gebildet und von bestimmten Zellen in der Haut, Aderhaut, Substantia nigra, Haaren etc. gespeichert wird, bestimmt im wesentlichen die Pigmentierung, d.h. die Färbung von Haut und Augen, Haaren und Federn. Menschen verschiedener Hautfarbe haben vergleichbare Melanozytenzahlen, die Farbunterschiede sind lediglich auf die Konzentration und Verteilung der Melanine zurückzuführen. So ist bei Sommersprossen, Leberflecken und dgl. die Melanozytenzahl zwar nicht erhöht, wohl aber die Melanin-Konzentration in diesen Gewebspartien. Das Fehlen von Melanin führt zum Albinismus.

Chemisch handelt es sich bei Melaninen um komplexe Aggregate chinoider Substanzen, die sich vom Indol ableiten und unter der Einwirkung bestimmter Enzyme, beispielsweise der Tyrosinase (einer Phenoloxidase), aus aromatischen Chromogenen entstehen. Dabei spielen Hydroxylierungen (zu Dihydroxyphenyl-L-alanin (Dopa)), Oxidationen (z.B. von Dopa zu Dopachinon) und Cyclisierungen eine wesentliche Rolle nach der folgenden Gleichung

Tyrosinase

Tyrosin ----------------> Dopa ------> (Zwischenprodukte) -----> Melanin

Die Art der bei der Melaninbildung entstehenden Zwischenprodukte ist noch nicht völlig geklärt, es soll sich dabei im wesentlichen um ortho-Chinone, Dopachrom, Indolchinon und Adrenochrom handeln (vgl. die nachstehende Tafel 1 "Melanin-Biosynthese").

## Tafel 1: Melanin-Biosynthese*

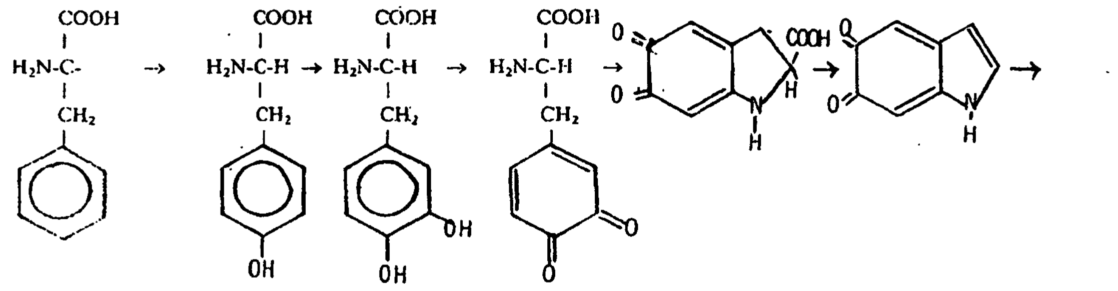

Phenylalanin Tyrosin DOPA

Phenylalanin Tyrosin DOPA

* Die Melanin-Synthese erfolgt in den Melanozyten der Haut. Die Anzahl der Melanozyten ist bei allen Menschen etwa gleich. Unterschiedliche Hautfarben entstehen durch verschieden starke Melanin-Produktion und Konzentration in den Zellen.

Man unterscheidet zwischen Eumelaninen und den in roten Haaren und Vogelfedem vorkommenden Phäomelaninen, die noch an Dopachinon gebundenes Cystein enthalten (Thomson, "Angew. Chemie" 86 (1974), 355-363). Melanin, ein ca. 10 bis 15 % Eiweiß enthaltendes Polymerisat (Melanoprotein) ist strukturell mit den Huminsäuren und Ligninen verwandt und bildet leicht freie Radikale, die wiederum als Radikalfänger dazu dienen können, die durch Sonnenstrahlung im Hautgewebe gebildeten schädlichen Radikale unschädlich zu machen.

Die Melanin synthetisierenden Enzyme (vgl. die nachstehende Tafel 2, in der die enzymatische Oxidation von Tyrosin zu Melanin in Gegenwart des Enzyms Tyrosinase dargestellt ist) liegen gewöhnlich in einem gehemmten Zustand vor. Durch Sonnen-, $\alpha$- oder Röntgenstrahlung wird diese Hemmung beseitigt, sodass sich mehr Melanin bildet. Umgekehrt kann die Bildung von Melanin beispielsweise durch Hydrochinonbenzylether auch künstlich gehemmt werden, sodass derartige Verbindungen zur Depigmentierung Verwendung finden können. Beim Albinismus ist die zu Melanin führende Reaktionssequenz blockiert, während die Ursache der Vitiligo (weiße Flekken auf der Haut) bis heute unbekannt ist.

**Tafel 2:** **Enzymatische Oxidation von Tyrosin zu Melanin**

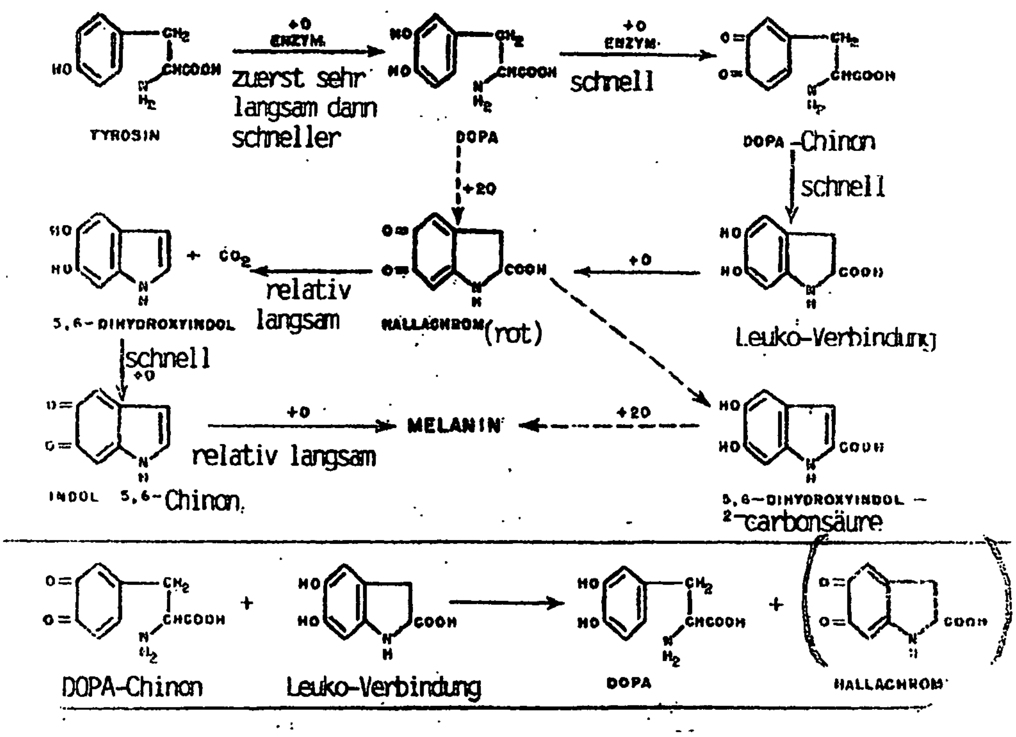


Ein Auftreten von ungleichmäßig braunen Stellen, meist an den Gliedmaßen und im Gesicht, von vorzugsweise alternden Menschen wird Melanose genannt. Melanine kommen auch in gut- und bösartigen Geschwulsten (Melanomen) und als pathologische Bestandteile im Harn vor. Das aus Harn isoliertes Melanin ist ein dunkelbraunes bis schwarzes Pulver, das sich bei 185 °C zersetzt.

Bei manchen Wirbeltieren, insbesondere Amphibien, Reptilien und Fischen, sind an der Ausbreitung von Melanin in der Haut die Hormone Melanotropin bzw. Melatonin als Gegenspieler beteiligt, indem sie eine Dilatation bzw. Kontraktion der Melanin führenden Zellen (Melanophoren) bewirken, sodass die Haut dunkler bzw. heller erscheint. Melanin findet sich auch im Skelett der Insekten und als färbender Bestandteil in der Tinte der Tintenfische.

Unter der Wirkung von Melanin wird bei Kaltblütern (Fischen, Amphibien, Reptilien) durch Expansion der Melanophoren und durch Dispersion der in diesen Zellen enthaltenen Melanine die Haut dunkler. Die antagonistische Wirkung des Hormons Melatonin macht diesen Vorgang reversibel, sodass beide Prozesse zusammen eine Farbanpassung an die Umwelt ermöglichen. Der eigentliche Wirkungsmechanismus des Melanins ist unbekannt, ebenso die physiologische Bedeutung für Vögel und Säugetiere, in denen es ebenfalls vorkommt. Melanin soll Hypocalcämie, Hyperlipämie und eine Steigerung der Schilddrüsenfunktion bewirken, in den Fettstoffwechsel eingreifen und möglicherweise auch die Dunkeladaption und die Resynthese des Sehpurpurs im Auge begünstigen sowie einen Einfluss auf die Wachstumsprozesse haben.

Melanine als Verursacher von unregelmäßigen braunen Farbflecken hauptsächlich an den Gliedmaßen und im Gesicht, die beim Menschen insbesondere mit zunehmendem Alter auftreten, sind jedoch unerwünscht, da man vermutet, dass sie unter bestimmten Umständen Vorstufen zu den gefürchteten bösartigen Melanomen darstellen. Sie können in verschiedenen Formen auftreten, wobei gutartige Pigmentflecken von malignen Melanomen nicht ganz leicht zu unterscheiden sind. Die einzige Art der Behandlung der letztgenannten ist ihre möglichst frühzeitige Entfernung, bevor diese metastasieren. Außerdem ist eine solche fleckenförmige Braunverfärbung der Haut häufig auch aus kosmetischen und ästhetischen Gründen unerwünscht.

Man ist daher seit langem auf der Suche nach Möglichkeiten, Melaninverfärbungen der Haut zu verhindern oder bereits eingetretene Verfärbungen zu vermindern oder zu beseitigen.

Es ist bereits bekannt, dass bestimmte chemische Verbindungen, beispielsweise die oben genannten Hydrochinonbenzylether, aber auch Gluconsäure (aufgrund ihrer Spurenelemente bindenden Wirkung), Dithiooctandiol (aufgrund seiner Tyrosin-Glycosylierungs-Hemmung), β-Carotine, Kojisäure, Arbutin sowie nicht näher definierte Oligopeptide eine die Melaninbildung (Melanogenese) hemmende Wirkung haben (hier als Whitening-Effekt bezeichnet). Dieser

"Whitening-Effekt" wird hauptsächlich durch Verbindungen bewirkt, welche die Tyrosinase-Aktivität regulieren und damit die Melanogenese verlangsamen. Eine lokale Pigmentierung der Hautoberfläche durch Melanin wird dadurch herabgesetzt bzw. gestoppt.

Aus "DATABASE CAPLUS AMERICAN CHEMICAL SOCIETY", 1989, XP002258107, gefunden im STN Database Accession no. 1989:121038 ist eine kosmetische Zusammensetzung mit "Whitening-Effekt" bekannt, die Dehydroessigsäure als Wirkstoff enthält. Nach den Angaben in dieser Druckschrift muss der Gehalt an Dehydroessigsäure 0,6 bis 5 Gew.-% betragen, um einen zufriedenstellenden Whitening-Effekt zu ergeben.

Die bisher bekannten Melanogenese-Hemmer sind jedoch in bezug auf ihre Wirksamkeit unbefriedigend. Aufgabe der Erfindung war es daher, wirksamere Melanogenese-Hemmer zu finden.

Es wurde nun gefunden, dass Enol-Lactone der nachstehend angegebenen allgemeinen Formel (I) und deren Tautomere mit den nachstehend angegebenen Formeln (Ia, Ib und Ic) sowie Salze, insbesondere Erdalkali-, Alkali- und Ammoniumsalze, sowie Derivate davon, besonders wirksame Melanogenese-Hemmer darstellen und einen bemerkenswerten Whitening-Effekt in kosmetischen Formulierungen ergeben.

Gegenstand der Erfindung sind kosmetische Zusammensetzungen mit einem ausgeprägten "Whitening-Effekt", die als Wirkstoff mindestens ein Enol-Lacton (nachstehend als "3-Acyl-3,4-dihydro-6-alkyl-2H-pyran-2,4-dion" oder abgekürzt als "ADAPD" bezeichnet) der nachstehend angegebenen allgemeinen Formel (I) und/oder eines oder mehrere seiner Tautomeren der Formeln (Ia - Ic) oder Salze, insbesondere Erdalkali-, Alkali- und/oder Ammoniumsalze derselben, und/oder an ihren OH-Gruppen modifizierte Derivate davon:

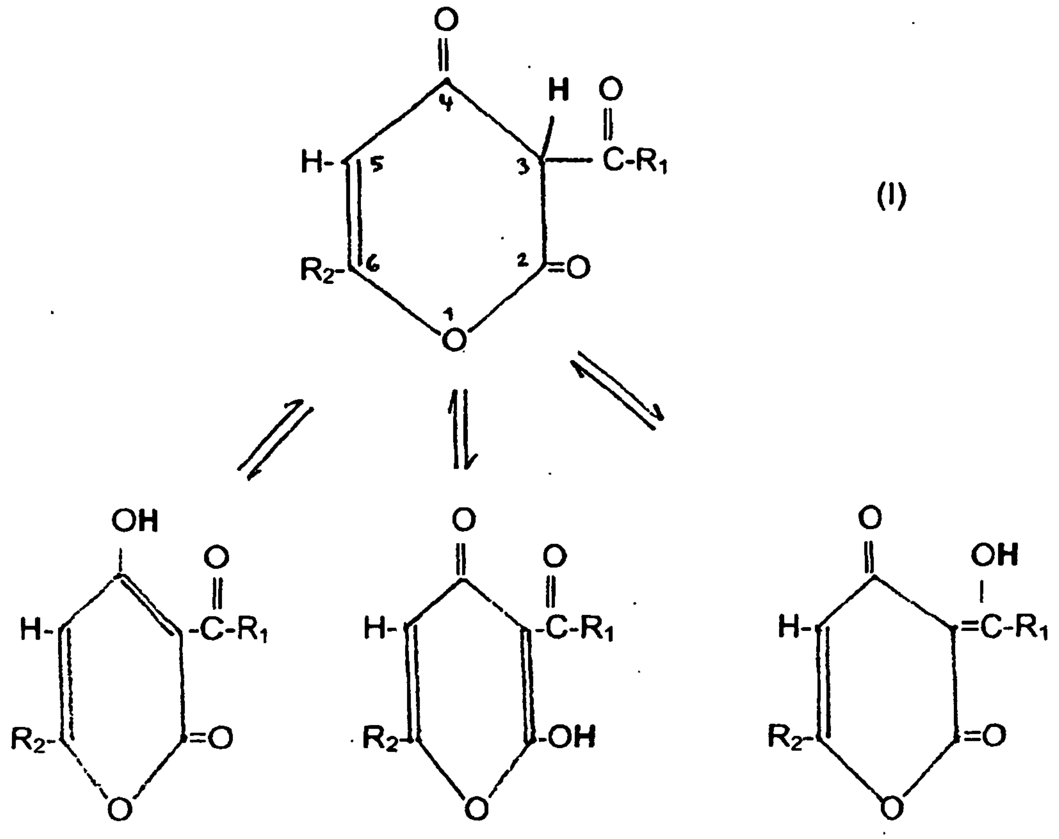

(I)

(Ia) (Ib) (Ic)

worin $R_1$ und $R_2$ gleich oder voneinander verschieden sein können, wobei $R_1$ für Phenyl, $C_{1-4}$-Alkylphenyl, insbesondere Benzyl, $C_{1-4}$-Alkyl, $C_{1-4}$-Alkenyl oder $C_{1-4}$-Alkinyl steht und $R_2$ für COOH oder COOR (worin R die für $R_1$ angegebenen Bedeutungen hat) oder für die gleichen Reste wie $R_1$ steht, und worin die OH-Gruppen gegebenenfalls teilweise oder vollständig durch Halogenidreste, insbesondere Cl und/oder Br, oder durch Oxyglycosid-, Thioglycosid- und/oder Aminoglycosid-Reste ersetzt sein können,

in einer Menge von 0,01 bis 0,5 Gew.-%, bezogen auf das Gewicht der kosmetischen Zusammensetzung, enthalten.

Ein solcher Whitening-Effekt, der mit den erfindungsgemäßen kosmetischen Zusammensetzungen erzielt werden kann, ist nicht nur anhand der Tyrosinase-Hemmung, sondern auch durch Bestimmung der Melanin-Produktions-Hemmung mit $B_{16}$-Melanom-Zellen aus Mäusen in Zellkulturversuchen nachweisbar. Der Nachweis des Whitening-Effektes beispielsweise einer ein Enol-Lacton einer der vorstehend angegebenen Formeln als Wirkstoff enthaltenden Hautcreme ist beispielsweise auf die weiter unten beschriebene Art und Weise möglich.

Besonders bevorzugt ist eine kosmetische Zusammensetzung, die als Wirkstoff 3-Acyl-3,4-dihydro-6-alkyl-2H-pyran-2,4-dion einer der Formeln (I) bzw. (Ia, Ib, Ic), worin $R_1$ und $R_2$ für $CH_3$ oder $C_2H_5$ stehen (nachstehend abgekürzt als "Methyl-ADAPD" bzw. als "Ethyl-ADAPD" bezeichnet), und/oder ein oder mehrere Salze, insbesondere Erdalkali-, Alkali- (speziell Natrium) und/oder Ammoniumsalze oder ein oder mehrere an einer oder mehreren OH-Gruppen substituierte Derivate desselben enthält.

Die erfindungsgemäße kosmetische Zusammensetzung kann auch als einzigen Wirkstoff oder zusätzlich zu den oben genannten Wirkstoffen mindestens einen solchen ADAPD-Wirkstoff der Formel (I) bzw. (Ia, Ib, Ic) enthalten, in dem die OH-Gruppen teilweise oder vollständig durch Halogenidreste, insbesondere Cl und/oder Br, oder durch Oxyglycosid-, Thioglycosid- und/oder Aminoglycosid-Reste ersetzt sind.

Die erfindungsgemäße kosmetische Zusammensetzung enthält den (die) oben genannten ADAPD-Wirkstoff (e) der Formel (I) und/oder der Formeln (Ia, Ib, Ic) oder Salze oder Derivate davon in einer Menge von 0,01 bis 0,5 Gew. %, besonders bevorzugt in einer Menge von 0,15 bis 0,5 Gew.-%, jeweils bezogen auf das Gesamtgewicht der kosmetischen Zusammensetzung.

Vorzugsweise enthalten die erfindungsgemäßen kosmetischen Zusammensetzungen der vorstehend beschriebenen Art als zusätzliche Additive Aldohexonsäuren, Ketohexonsäuren, Weinsäure, Citronensäure und/oder Ascorbinsäure, vorzugsweise in einer Menge von 0,01 bis 5,0 Gew.-%, insbesondere von 0,1 bis 0,5 Gew.-%, bezogen auf das Gesamtgewicht der kosmetischen Zusammensetzung.

Die erfindungsgemäßen kosmetischen Zusammensetzungen können gemäß einer weiteren bevorzugten Ausgestaltung als weitere Additive S-haltige Aminosäuren und/oder S-haltige Oligopeptide, insbesondere Glutathione, vorzugsweise in einer Menge von 0,01 bis 3,0 Gew.-%, insbesondere in einer Menge von 0,1 bis 0,5 Gew.-%, bezogen auf das Gesamtgewicht der kosmetischen Zusammensetzung, enthalten.

Die erfindungsgemäßen kosmetischen Zusammensetzungen können außerdem einen oder mehrere weitere Wirkstoffe neben den oben genannten ADAPD-Wirkstoffen der Formeln I, Ia, Ib und Ic, vorzugsweise keratolytisch wirksame Verbindungen, insbesondere Keratase und/oder $\alpha$-Hydroxysäuren (AHA-Säuren), speziell Rubiginsäure, Hydracetsäure, Comensäure, Chelidonsäure und/oder Mekonsäure, vorzugsweise in einer Menge von 0,01 bis 5,0 Gew.-%, insbesondere von 0,1 bis 0,5 Gew.-%, bezogen auf das Gesamtgewicht der kosmetischen Zusammensetzung, enthalten.

Außerdem können die erfindungsgemäßen kosmetischen Zusammensetzungen Sorbit, Harnstoff, ein Succinat, Ethanol, Dexpanthenol sowie übliche kosmetische Zusätze, Hilfsstoffe, Träger und dgl.,
sowie übliche kosmetische Zusätze, Hilfsstoffe, Träger und dgl. in den üblichen Mengen, beispielsweise in einer Gesamtmenge von bis zu 50 Gew.%, vorzugsweise von 3 bis 7 Gew.-%, bezogen auf das Gesamtgewicht der Zusammensetzung, enthalten.

Die erfindungsgemäße kosmetische Zusammensetzung liegt vorzugsweise in Form einer Lotion, einer Creme, einer Salbe, einer Lösung, eines Gels oder eines Sprays vor.

Gegenstand der Erfindung ist ferner ein Verfahren zur Herstellung der vorstehend beschriebenen erfindungsgemäßen kosmetischen Zusammensetzungen, bei dem besonders auf den pH-Wert der Lösung bzw. Dispersion der übrigen Bestandteile der kosmetischen Zusammensetzung, die zuerst hergestellt wird, vor oder beim Zusatz der oben genannten ADAPD-Wirkstoffe geachtet werden muss. Wesentlich ist dabei, dass der pH-Wert der Lösung bzw. Dispersion immer $\leq$ 7,0, vorzugsweise 6,0 bis 7,0, insbesondere 6,3 bis 6,7, beträgt.

Vorzugsweise wird (werden) der (die) ADAPD-Wirkstoff(e) (der Formel I bzw. Analoge und Derivate davon) immer erst gegen Ende der Operationen bei pH-Werten zwischen 6 und 7, vorzugsweise zwischen 6,3 und 6,7, zugegeben, woran sich eine Homogensierung anschließt. Der gesamte kosmetische Ansatz wird dann - zur Herstellung einer Creme - gegebenenfalls nach dem Entlüften, Homogenisieren und Kaltrühren, über einen Salbenstuhl laufen gelassen.

Bei der Herstellung der erfindungsgemäß verwendeten ADAPD-Wirkstoffe der Formel I hat sich gezeigt, dass die ADAPD-Wirkstoffe (Formel I, $R_1 = R_2 = CH_3$ oder $C_2H_5$), die als Rückstand bei der Umsetzung von Essigsäureethylester oder Propionsäureethylester mit Natrium als Rückstand anfallen, ohne weitere Reinigung eingesetzt werden können. Im Gegensatz dazu müssen die Ausgangsstoffe, der Essigsäureethylester und der Propionsäureethylester, intensiv gereinigt werden, bevor sie eingesetzt werden.

Als ADAPD-Wirkstoff der Formel I bzw. Derivate davon mit Whitening-Effekt kommen auch die bei der Umsetzung von $CH_3$-ADAPD mit Chlor entstehende Verbindung mit einem Schmelzpunkt von 93 °C (vgl. Oppenheim, Precht, B. 9, 1101) sowie die bei der Umsetzung mit Brom entstehende Verbindung mit einem Schmelzpunkt von 137 °C (aus Methanol) (vgl. Oppenheim, Precht, B. 9, 1101; Perkin, Bernhart, B. 17, 1524) in Betracht, wobei die geringe Löslichkeit des Bromderivats zusätzliche Maßnahmen erforderlich macht. Bei der Einwirkung von $PCl_5$ + $POCl_3$ auf $CH_3$-ADAPD

entsteht das entsprechende Chlorid $C_8H_6O_2Cl_2$ mit einem Schmelzpunkt von 101 °C (aus Ethanol), das ebenfalls als Wirkstoff von Interesse ist, insbesondere im Gemisch mit $CH_3$-ADAPD (vgl. Feist, A. 257, 283-285, und Oppenheim, Precht, B. 9, 1100).

Gegenstand der Erfindung ist außerdem die Verwendung der vorstehend beschriebenen erfindungsgemäßen kosmetischen Zusammensetzungen, insbesondere der darin enthaltenen ADAPD-Wirkstoffe der Formeln I, Ia, Ib und/oder Ic, zur topischen Behandlung von braunen Hautflecken bei Menschen und Tieren.

Der Whitening-Effekt von erfindungsgemäßen kosmetischen Zusammensetzungen kann wie in den nachstehenden Beispielen für erfindungsgemäße Hautcremes, die den Wirkstoff ADAPD enthalten, beschrieben, bestimmt werden.

Beispiel 1: Handcreme mit Methyl-ADAPD als Wirkstoff

Nach folgender Vorschrift wurde eine Lanolin enthaltende Handcreme formuliert, welcher der Wirkstoff Methyl-ADAPD in der nachstehend angegeben Menge einverleibt wurde. Die Endzusammensetzung der Creme-Formulierung war wie folgt:

| | |
|---|---|
| lösliches Lanolin-Derivat | 1,0 Gew.-% |
| acetylierte Lanolinalkohole | 5,0 Gew.-% |
| flüssiger Multisterol-Extrakt | 5,0 Gew.-% |
| Vaseline | 5,0 Gew.-% |
| Polyvinylalkohol-Gel | 0,75 Gew.-% |
| 10 %iges NaOH | 2,25 Gew.-% |
| ethoxylierte Fettamine | 3,75 Gew.-% |
| Methyl-ADAPD (Formel I, $R_1$, $R_2$ = $CH_3$) | 0,4 Gew.-% |
| Parfümöl | 0,3 Gew.-% |
| Wasser, bidest. | ad 100,00 Gew.-% |

Zur Herstellung dieser Handcreme wurden die Fettphasen-Bestandteile und Vaseline auf etwa 75 °C erwärmt. Gleichzeitig wurde das PVA-Gel in heißem Wasser von etwa 80 °C dispergiert und allmählich gelöst. Zu der dabei erhaltenen Dispersion wurden das ethoxylierte Fettamin (mit etwa 25 EO-Einheiten) und gegebenenfalls noch ein Emulgator zugegeben, danach wurde die vorher angesetzte erwärmte Fettphase darin emulgiert. Nach 5-minütigem Rühren bei der Mischungstemperatur wurde auf 60 °C abgekühlt, dann mit 10 %iger Natronlauge versetzt und weiter bis auf 40 °C gekühlt. Nach einer pH-Messung und Einstellung auf einen pH-Wert von 6 bis 7, vorzugsweise 6,7, wurden dann unter langsamem Rühren 0,4 Gew.-% Methyl-ADADP sowie das Parfümöl zugesetzt. Der Ansatz wurde gut homogenisiert.

Die Handcreme war stabil konfektioniert und zeigte eine verbessernde Hautkonditionierung, verglichen mit einem identischen Ansatz ohne den Wirkstoff Methyl-ADAPD.

Nachweis des Whitening-Effekts für die vorstehend beschriebene Handcreme

Nach Zerstörung der Emulsion der Handcreme durch Wasserzusatz und Erhitzen auf 80 °C wurde die wässrige Phase abgetrennt, im Vakuum eingeengt und der Zentrifugenrückstand wurde gefriergetrocknet.

Definierte Mengen des dabei erhaltenen Lyophilisats wurden in entionisiertem Wasser gelöst und nach den Angaben in dem weiter unten beschriebenen Testverfahren behandelt.

Tabelle 1

| Wirkstoff*-Gehalt in mg/ml Lösung | Anzahl der Zellen | Relativwert | Whitening-Effekt |
|---|---|---|---|
| 1,25 | $82x10^4$ | 0,92 | + |
| 2,5 | $79x10^4$ | 0,86 | +++ |
| Kontrolle (0) | $89x10^4$ | 1,00 | - |

* Wirkstoff = Methyl-ADAPD (Formel I mit $R_1$, $R_2$ = Methyl)

Bestimmung der Tyrosinase-Inhibitorrate bei Verwendung der nachstehend angegebenen Wirkstoff-Gehalte (Wirkstoff = Methyl-ADAPD) in mg/ml:

Tabelle 2

| Wirkstoff-Gehalt* (in mg/ml Lösung) | Tyrosinase-Inhibitorrate | Extinktion |
|---|---|---|
| 5 | 99,1 % | 0,006 |
| 0,5 | 84 % | 0,078 |
| Kontrolle (0) | | 0,004 |

* Wirkstoff = Methyl-ADAPD

Testverfahren zur Bestimmung des Whitening-Effekts von Methyl-ADAPD

$B_{16}$-Melanom-Zellen von Mäusen wurden überimpft und kultiviert 6 Tage lang mit Testproben bei 37 °C, 5 % $CO_2$ + 95 % Luft, wobei das Kulturmedium am dritten Tag gewechselt wurde.
Die grobe Beurteilung der Whitening-Effekte wurde vorgenommen nach der Abtrennung der Zellen unter Verwendung von 6 ml EDTA-Lösung und Auszählen der Zellen, z.B. Tabelle 1 und Tabelle 3 (Beurteilungskriterien auf S. 15).

Beurteilungsmethode

a) Skala zur Bewertung des Whitening-Effekts (Melanin-Hemmung)

| | |
|---|---|
| wie die Blindkontrolle | - |
| leichter Whitening-Effekt | + |
| ausgeprägter Whitening-Effekt | ++ |
| starker Whitening-Effekt | +++ |

Reihenfolge der Testverfahrensmaßnahmen:

1. Herstellung und Sterilisierung eines Test-Kulturmediums
2. Herstellung einer Zellenflotationslösung
3. Einstellung des pH-Wertes des Kulturmediums
4. Ausstreichen des Kulturmediums auf einer Petrischale
5. Einimpfen von $B_{16}$-Melanom-Zellen von Mäusen
6. Kultivieren in einem Thermostaten (37 °C, 5 % $CO_2$, 95 % Luft)
7. Auswechseln des Kulturmediums am 3. Tag
8. Kultivieren in dem Thermostaten (37 °C, 5 % $CO_2$, 95 % Luft)

Tabelle 3 - Beispiel für einen $B_{16}$ Melanom-Zellen-Test

| Probe | Menge | Whitening-Effekt | Anzahl der Zellen |
|---|---|---|---|
| A | 0,500 | + | ± |
| | 0,200 | + | ± |
| B | 0,500 | ± | ± |
| | 0,200 | ± | ± |
| (I)* | 0,250 | +++ | - |
| | 0,200 | ++ | ± |
| Kontrolle | 0 | ± | |

* mit $CH_3$-ADAPD

Beurteilungskriterien

1. Ausmaß des Whitening-Effekts

| | |
|---|---|
| ± | nahezu gleiche Dichte wie die Kontrollprobe |
| + | geringer Whitening-Effekt gegenüber der Kontrolle |
| ++ | erkennbarer Whitening-Effekt gegenüber der Kontrolle |
| +++ | weiß-grauweiß (nicht mehr schwarzes Pigment) |

2. Anzahl der Zellen

| | |
|---|---|
| ± | nahezu gleiche Anzahl wie die Kontrolle |
| - | 2/3 der Anzahl der Kontrolle |
| -- | 1/2 der Anzahl der Kontrolle |
| --- | 1/3 der Anzahl der Kontrolle |

Tabelle 4: Tyrosinase-Inhibitorrate

| | | |
|---|---|---|
| Charge A: | 0,25 mg/ml | 72,3 % |
| | 0,1 mg/ml | 24,1 % |
| Charge B: | 0,5 mg/ml | 96,4 % |
| | 0,2 mg/ml | 43,6 % |
| Charge (I) | 0,25 mg/ml | 67,2 % |
| | 0,1 mg/ml | 17,7 % |

Padberg-Test mit der wie vorstehend angegeben hergestellten Handcreme (enthaltend Methyl-ADAPD als Wirkstoff)

Beschreibung der Padberg-Methode

Im Padberg-Test wird die Rauigkeit einer Haut anhand derjenigen Menge Methylenblau bewertet, die in Abhängigkeit von den Hautkonditionen von der Haut absorbiert wird. Studien mit "subjektiv rauer Haut" bzw. mit "durch Tenside geschädiger Haut" ergaben, dass die absorbierte Methylenblau-Menge der Hautrauigkeit proportional ist. Gute Haut bzw. gut gepflegte Haut absorbiert demgegenüber nur eine geringe Menge Methylenblau.

Zu dem Test wurden 10 Probandinnen im Alter von 40 bis 66 Jahren herangezogen. Die Versuchspersonen stellten sich freiwillig für diesen Test zur Verfügung. Sie wurden angewiesen, drei Tage vor Beginn des Tests und während der gesamten Testdauer keine anderen Kosmetika auf den Testarealen (Unterarme) zu verwenden. Bei jeder Probandin wurden sechs Testfelder auf beiden Unterarm-Innenseiten (also drei je Arm) markiert. Die Areale waren so zugewiesen, dass die Produkte gleich häufig bei den zehn Probandinnen auf die sechs Testfelder verteilt waren (gleichförmige Permutation). Neben den fünf Emulsionen wurde ein Leerfeld mitgeprüft. Nachdem die Leerwerte aller Areale nach beiden Methoden bestimmt waren, wendeten die Probandinnen die Produkte selbst zu Hause in einem Zeitraum von 14 Tagen morgens und abends an. Danach wurde 4 Stunden nach der letzten Anwendung erneut nach Padberg geprüft.

Zur Durchführung des Padberg-Tests werden folgende Schritte angewendet:

(1) Markierung der Testfelder
(2) Vorbehandlung der Testfelder mit einer 1 %igen Lösung eines nichtionischen Tensids in Wasser
(3) Nachwaschen mit Leitungswasser von 35 °C
(4) Abtupfen der Testfelder
(5) nach 30-minütigem Akklimatisieren der Probanden bei 22 °C und 60% relativer Luftfeuchte
(6) Auftragen von 20 µl Farblösung (die Farblösung bestand aus 0,5 %igem Methylenblau (20 %) und 80 % einer 1 %igen Lösung eines nichtionischen Tensids) und 30 s verteilen lassen

(7) Antrocknenlassen der Farbe für 30 s
(8) Entfernen des Farbübschusses mit 2 ml einer 0,25 %igen nichtionischen Tensidlösung
(9) Extraktion mit 2 x 2 ml einer Mischung aus:

2,0 % Natriumlaurylsulfat
48,0 % Wasser
50,0 % Isopropylalkohol
jeweils nach 60 s

(10) Messen des Eluats im Spektralphotometer bei 660 nm gegen Luft
(11) Ergebnis = Extinktion Haut (Ausgangswert)
(12) es erfolgte nun die Ausgabe der Produkte mit der Anweisung, diese 14 Tage lang morgens und abends auf den vorgesehnenen markierten Stellen aufzutragen.
Zuvor wurde sichergestellt, dass 3 Tage vor Beginn der Prüfung und während der gesamten Prüfdauer keine anderen kosmetischen Produkte auf den Testarealen angewendet wurden.
(13) Wiederholung der Schritte (1) bis (9) am 15. Tag. Die letzte Anwendung vor dem Test erfolgte 4 Stunden zuvor.
Ergebnis = Extinktion nach der Anwendung
(14)

$$\frac{\text{Extinktion nach der Anwendung}}{\text{Extinktion Hautausgangswert}} \times 100 = \text{Rauigkeit (\%)}$$

Diskussion der Padberg-Methode

Die Padberg-Methode liefert Daten über die Adsorption von Methylenblau auf der Haut. Je nach Hautzustand stehen dem Farbstoff mehr oder weniger Bindungsstellen auf der Haut zur Verfügung. Jahrelange Erfahrungen haben immer wieder bestätigt, dass subjektiv "raue" Haut oder beispielsweise durch Detergentien geschädigte Haut mehr Bindungsstellen für Methylenblau aufweist, und zwar in Abhängigkeit vom Schädigungsgrad. Umgekehrt zeigt eine mit Cremes verbesserte Haut eine Abnahme der Bindungsstellen, und proportional zur Anwendungsdauer. Die Differenzierung bei verschiedenen Produkten ist sehr gut und verlässlich; auch hier wird stets eine Korrelierung mit der subjektiven Begutachtung erzielt.

Ergebnisse des Padberg-Tests:

Die Fotometer-Werte, ausgedrückt in Prozent des Wertes bei behandelter Haut und bezogen auf den Ausgangszustand, sind in der folgenden Tabelle 5 zusammengefasst. In der Tabelle sind auch die errechneten Mittelwerte angegeben.
Die Ergebnisse zeigen, dass mit den Testemulsionen eine Glättung der Haut erzielbar ist.

Tabelle 5 Rauigkeitstest nach der Padberg-Methode mit Wasser/Öl-Emulsionen mit und ohne Zusatz von Tripeptiden (Anwendungsdauer 14 Tage)

| Alter der Probanden | Plazebo | Wasser-Öl-Emulsionen der Erfindung | |
|---|---|---|---|
| 46 | 81,0 | 50,1 | 60,0 |
| 41 | 91,0 | 64,4 | 64, 9 |
| 47 | 80,0 | 76,5 | 40,0 |
| 50 | 76,6 | 33,0 | 41,2 |
| 51 | 75,3 | 56,8 | 70,0 |
| 40 | 95,0 | 37,5 | 40,5 |
| 65 | 89,0 | 72,5 | 68,0 |
| 66 | 97,8 | 57,8 | 55,0 |
| 62 | 92,6 | 70,0 | 60,0 |

Tabelle fortgesetzt

| Alter der Probanden | Plazebo | Wasser-Öl-Emulsionen der Erfindung | |
|---|---|---|---|
| 45 | 76,9 | 60,7 | 62,7 |
| Mittelwert | 85,6 | 58,2 | 56,4 |

Klinisch-allergologische Prüfung der wie oben beschrieben hergestellten Handcreme (enthaltend den Wirkstoff Methyl-ADAPD) im Patch-Test am Menschen

Zusammenfassung: der Wirkstoff Methyl-ADAPD ist im Patch-Test auf allergisierendes Potenzial geprüft worden. In einer präklinischen Prüfung wurde die lokale Verträglichkeit abgeklärt. Diese Prüfung zeigte keine Bedenken für den Einsatz der Produkts in einer klinischen Prüfung am Menschen.

Die klinische Prüfung im Patch-Test ergab für die Frage des Einsatzes der wie oben beschrieben hergestellten Handcreme (enthaltend den Wirkstoff Methyl-ADAPD) im Gesicht des Menschen vom allergologischen Standpunkt keine Bedenken, da bei den 60 geprüften Probanden dahingehend keinerlei Reaktion auftrat.

Testmethode: Diese wurde durchgeführt unter den Bedingungen eines epikutanen Läppchentests. Eine kleine Menge Testsubstanz wird auf hygroskopiche Testplättchen aufgebracht und mittels Heftpflaster auf der Rückenhaut der Patienten befestigt, die wegen des Verdachtes auf das Vorliegen einer Kontaktallergie im allergologischen Labor der Klinik getestet wurden. Nach 24 Stunden wurden die mit der Testsubstanz beschickten Plättchen entfernt und die örtliche Hautreaktion an der Anliegestelle der Testplättchen wurde eine halbe Stunde nach Abnahme der Testpflaster abgelesen. Weitere Ablesungen erfolgten nach 48 h und 72 h. Die Ergebnisse sind in der nachstehenden Tabelle 6 zusammengefasst.

Bewertung:

| | |
|---|---|
| 0 | negativ |
| (-) | fraglich positiv, |
| + | schwach positiv, |
| ++ | positiv, |
| +++ | stark positiv |

Die Prüfung erfolgt unter einer extremen Belastung, die unter normalen Bedingungen nicht gegeben ist.

Tabelle 6: Ergebnisse des PATCH-Tests

| Patient Nr. | Testreaktion auf andere Stoffe | Reaktion der Methyl-ADAPD enthaltenden Handcreme nach 24 h | nach 48 h | nach 72 h |
|---|---|---|---|---|
| 1 | p-Phenylendiamin | 0 | 0 | 0 |
| 2 | Kaliumbichromat | 0 | 0 | 0 |
| 3 | | 0 | 0 | 0 |
| 4 | | 0 | 0 | 0 |
| 5 | | 0 | 0 | 0 |
| 6 | | 0 | 0 | 0 |
| 7 | | 0 | 0 | 0 |
| 8 | | 0 | 0 | 0 |
| 9 | | 0 | 0 | 0 |
| 10 | | 0 | 0 | 0 |
| 11 | Zimtaldehyd | 0 | 0 | 0 |
| 12 | Kolophonium | 0 | 0 | 0 |
| 13 | | 0 | 0 | 0 |
| 14 | Nickelsulfat | 0 | 0 | 0 |
| 15 | | 0 | 0 | 0 |
| 16 | Epoxidharz | 0 | 0 | 0 |

Tabelle fortgesetzt

| Patient Nr. | Testreaktion auf andere Stoffe | Reaktion der Methyl-ADAPD enthaltenden Handcreme | | |
|---|---|---|---|---|
| | | nach 24 h | nach 48 h | nach 72 h |
| 17 | Eucerin | 0 | 0 | 0 |
| 18 | Perubalsam | 0 | 0 | 0 |
| 19 | | 0 | 0 | 0 |
| 20 | | 0 | 0 | 0 |
| 21 | | 0 | 0 | 0 |
| 22 | Formaldehyd | 0 | 0 | 0 |
| 23 | | 0 | 0 | 0 |
| 24 | | 0 | 0 | 0 |
| 25 | Kobaltchlorid | 0 | 0 | 0 |
| 26 | Chloramphenicol | 0 | 0 | 0 |
| 27 | Quecksilber | 0 | 0 | 0 |
| 28 | | 0 | 0 | 0 |
| 29 | | 0 | 0 | 0 |
| 30 | | 0 | 0 | 0 |
| 31 | Lanette N | 0 | 0 | 0 |
| 32 | Bufexamac | 0 | 0 | 0 |
| 33 | | 0 | 0 | 0 |
| 34 | | 0 | 0 | 0 |
| 35 | | 0 | 0 | 0 |
| 36 | Formaldehyd | 0 | 0 | 0 |
| 37 | | 0 | 0 | 0 |
| 38 | | 0 | 0 | 0 |
| 39 | | 0 | 0 | 0 |
| 40 | Hydroxycitronellal | 0 | 0 | 0 |
| 41 | Abitol | 0 | 0 | 0 |
| 42 | Kolophonium | 0 | 0 | 0 |
| 43 | | 0 | 0 | 0 |
| 44 | | 0 | 0 | 0 |
| 45 | | 0 | 0 | 0 |
| 46 | Formaldehyd | 0 | 0 | 0 |
| 47 | | 0 | 0 | 0 |
| 48 | | 0 | 0 | 0 |
| 49 | Clotriazol | 0 | 0 | 0 |
| 50-60 | | 0 | 0 | 0 |

Ergebnisse: Die den Wirkstoff Methyl-ADAPD enthaltende Handcreme wurde von allen 60 Patienten im Epikutantest reaktionslos vertragen bei unverdünnter Anwendung.

Wie aus dem Testprotokoll hervorgeht, erwiesen sich 20 der 60 getesteten Personen als kontaktallergisch gegenüber verschiedenen Allergenen des Alltags und des Berufslebens.

Herstellung des erfindungsgemäß verwendbaren Wirkstoffs Ethyl-ADAPD (Formel I mit $R_1$, $R_2$ = Ethyl)

Die Synthese des Wirkstoffes Ethyl-ADAPD wird, wie in der nachstehenden Tafel 3 gezeigt, hergestellt aus

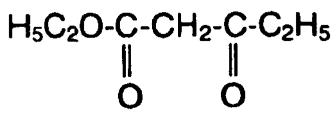

(ähnlich der bekannten Acetessigester-Synthese aus Essigester und Nebenreaktion):

**Tafel 3:** **Herstellung des Ethyl-Homologons der Formel I, worin $R_1$, $R_2$ = $C_2H_5$ (Ethyl-ADAPD) analog zur Bildung von 3-Acetyl-3,4-dihydro-6-methyl-2H-pyran-2,4-dion (Methyl-ADAPD)**

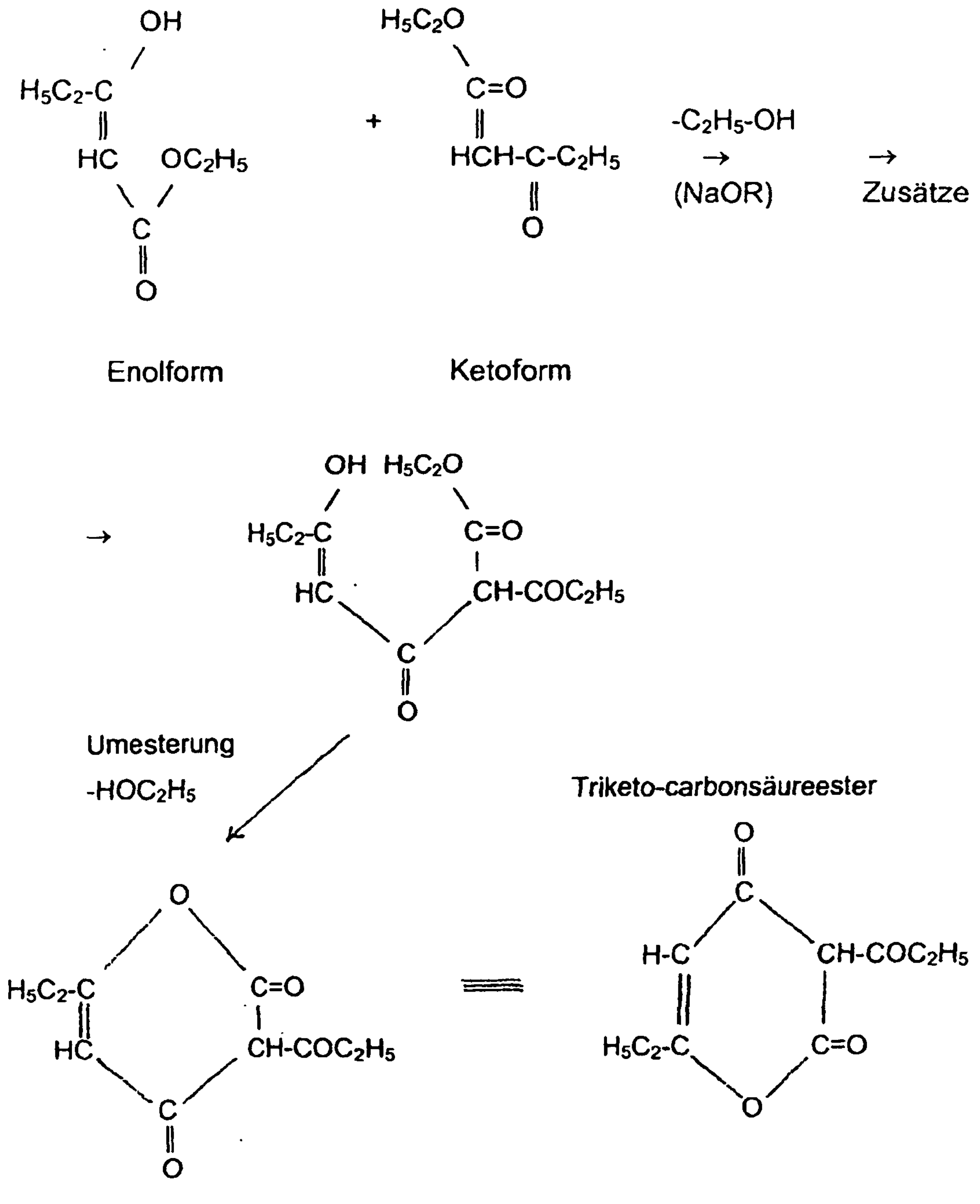


Enolform

Ketoform

Triketo-carbonsäureester

Ethylhomologon gemäß Formel I

3-Propionyl-3,4-dihydro-6-ethyl-2H-pyran-2,4-dion

Formel I ($R_1$, $R_2$ = $C_2H_5$)

(Ethyl-ADAPD)

Beispiel 2: Kosmetisches Additiv CYTOCATALYZER IKD*, enthaltend $CH_3$-ADAPD plus Chlor-behandeltes $CH_3$-ADAPD (F. 93°C)**

In die CYTOCATALYZER-Lösung von pH 6,6 werden unter Rühren 0,3 % $CH_3$-ADAPD plus 0,1 % der Chlor-Verbindung eingetragen, wobei der pH-Wert bei 6,6 gehalten wird.

Der mit dieser Lösung durchgeführt Melanoma B16-Test ergab den Wert +++.

* beschrieben in "The BI-MONTHLY JOURNAL OF BWW Society 2003, HTTP:\\www.BWW Society. Org\Journal\html 2003

** Oppenheim, Precht, B. 9, 1101

**Patentansprüche**

1. Kosmetische Zusammensetzung mit "Whitening-Effekt", die als Wirkstoff mindestens ein Enol-Lacton der nachstehend angegebenen allgemeinen Formel (I) und/oder eines oder mehrere seiner Tautomeren der Formeln (Ia-Ic)

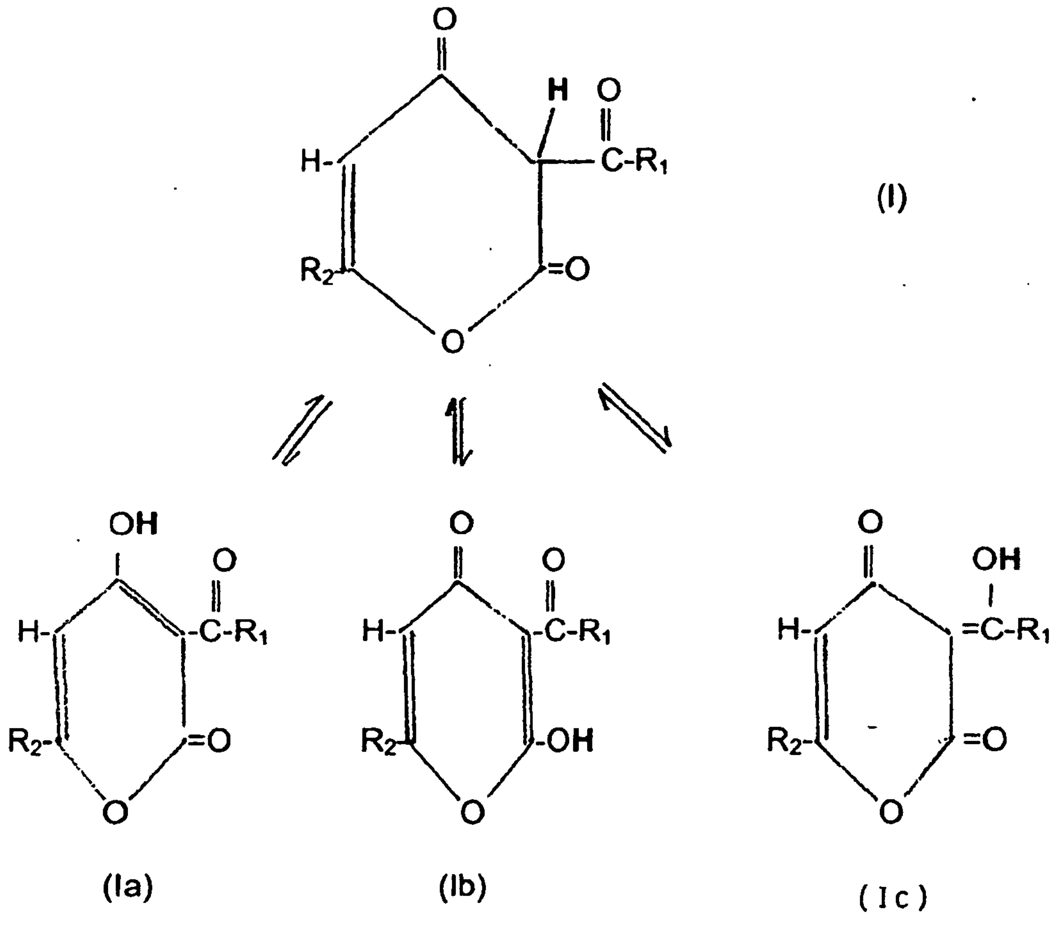

(I)

(Ia) (Ib) (Ic)

worin
$R_1$ und $R_2$ gleich oder voneinander sind, wobei
$R_1$ für Phenyl, $C_{1-4}$-Alkyl-phenyl, insbesondere Benzyl, $C_{1-4}$-Alkyl, $C_{1-4}$-Alkenyl oder $C_{1-4}$-Alkinyl steht, und
$R_2$ für COOH oder COOR (worin R die für $R_1$ angegebenen Bedeutungen hat) oder für die gleichen Reste wie $R_1$ steht, und
worin die OH-Gruppen gegebenenfalls teilweise oder vollständig durch Halogenidreste, insbesondere Cl und/oder Br, oder durch Oxyglycosid-, Thioglycosid- und/oder Aminoglycosid-Reste ersetzt sein können,
und/oder ein oder mehrere Salze, insbesondere Erdalkali-, Alkali- und/oder Ammoniumsalze, davon
in einer Menge von 0,01 bis 0,5 Gew.-%, bezogen auf das Gesamtgewicht der kosmetischen Zusammensetzung,

enthält.

2. Kosmetische Zusammensetzung nach Anspruch 1, **dadurch gekennzeichnet, dass** sie als Wirkstoff mindestens ein Enol-Lacton der in Anspruch 1 angegebenen Formeln (I), (Ia), (Ib) und (Ic) enthält, worin $R_1$ und $R_2$ gleich oder verschieden sind und für $CH_3$ oder $C_2H_5$ stehen.

3. Kosmetische Zusammensetzung nach einem der Ansprüche 1 bis 2, **dadurch gekennzeichnet, dass** sie als Wirkstoff 3-Acyl-3,4-dihydro-6-alkyl-2H-pyran-2,4-dion (ADAPD) der Formel (I) bzw. (Ia, Ib, Ic), worin $R_1$ und $R_2$ für $CH_3$ stehen (Methyl-ADAPD) oder für $C_2H_5$ stehen (Ethyl-ADAPD), oder ein Salz, insbesondere ein Natriumsalz, desselben enthält.

4. Kosmetische Zusammensetzung nach einem der Ansprüche 1 bis 3, **dadurch gekennzeichnet, dass** sie den (die) Wirkstoff(e) der Formel (I) bzw. (Ia, Ib, Ic) in einer Menge von 0,15 bis 0,5 Gew.-%, bezogen auf das Gesamtgewicht der kosmetischen Zusammensetzung, enthält.

5. Kosmetische Zusammensetzung nach einem der Ansprüche 1 bis 4, **dadurch gekennzeichnet, dass** sie als zusätzliche(s) Additiv(e) Aldohexonsäuren, Ketohexonsäuren, Weinsäure, Citronensäure und/oder Ascorbinsäure enthält.

6. Kosmetische Zusammensetzung nach Anspruch 5, **dadurch gekennzeichnet, dass** sie die zusätzlichen Additive in einer Menge von 0,01 bis 5 Gew.-%, vorzugsweise in einer Menge von 0,1 bis 0,5 Gew.-%, bezogen auf das Gesamtgewicht der kosmetischen Zusammensetzung, enthält.

7. Kosmetische Zusammensetzung nach einem der Ansprüche 1 bis 6, **dadurch gekennzeichnet, dass** sie als weitere (s) Additiv(e) S-haltige Aminosäuren und/oder S-haltige Oligopeptide, insbesondere Glutathione, enthält.

8. Kosmetische Zusammensetzung nach Anspruch 7, **dadurch gekennzeichnet, dass** sie die weiteren Additive in einer Menge von 0,01 bis 3,0 Gew.-%, vorzugsweise in einer Menge von 0,1 bis 0,5 Gew.-%, bezogen auf das Gesamtgewicht der kosmetischen Zusammensetzung, enthält.

9. Kosmetische Zusammensetzung nach einem der Ansprüche 1 bis 8, **dadurch gekennzeichnet, dass** sie als weitere (n) Wirkstoff(e) keratolytisch wirksame Verbindungen, insbesondere Keratase und/oder $\alpha$-Hydroxysäuren (AHA-Säuren), enthält.

10. Kosmetische Zusammensetzung nach Anspruch 9, **dadurch gekennzeichnet, dass** sie als weitere Wirkstoffe Rubiginsäure, Hydracetsäure, Comensäure, Chelidonsäure und/oder Mekonsäure enthält.

11. Kosmetische Zusammensetzung nach Anspruch 9 oder 10, **dadurch gekennzeichnet, dass** sie den (die) weiteren Wirkstoff(e) in einer Menge von 0,01 bis 5,0 Gew.-%, vorzugsweise in einer Menge von 0,1 bis 0,5 Gew.-%, bezogen auf das Gesamtgewicht der kosmetischen Zusammensetzung, enthält.

12. Kosmetische Zusammensetzung nach einem der Ansprüche 1 bis 11, **dadurch gekennzeichnet, dass** sie außerdem Sorbitol, Harnstoff, ein Succinat, Ethanol, Dexpanthenol sowie übliche kosmetische Zusätze, Hüfsstoffe, Träger und dgl. enthält.

13. Kosmetische Zusammensetzung nach einem der Ansprüche 1 bis 12, **dadurch gekennzeichnet, dass** sie in Form einer Lotion, einer Creme, einer Salbe, einer Lösung, eines Gels oder eines Sprays vorliegt.

14. Verfahren zur Herstellung der kosmetischen Zusammensetzung nach den Ansprüchen 1 bis 13, **dadurch gekennzeichnet, dass** man einen oder mehrere Wirkstoffe der Formel (I) und/oder Tautomere und/oder Derivate davon einer in an sich bekannter Weise hergestellten Lösung oder Dispersion der übrigen Bestandteile der kosmetischen Zusammensetzung zusetzt, nachdem die Lösung bzw. Dispersion auf einen pH-Wert zwischen 6 und 7, insbesondere zwischen 6,3 und 6,7, eingestellt worden ist, und das dabei erhaltene Gemisch dann in üblicher Weise entlüftet, homogenisiert, kalt rührt und gegebenenfalls über einen Salbenstuhl laufen lässt.

15. Verwendung der kosmetischen Zusammensetzung nach den Ansprüchen 1 bis 14, insbesondere der darin genannten ADAPD-Wirkstoffe der allgemeinen Formeln I, Ia, Ib, Ic, speziell von Methyl-ADAPD und/oder Ethyl-ADAPD, zur Verhinderung der Bildung bzw. zur Abschwächung oder Entfernung von braunen Hautflecken bei Mensch und

Tier.

## Claims

1. A cosmetic composition having a "whitening effect" which composition contains, as an active ingredient, at least one enol-lactone having the following general formula (I) and/or one or more of its tautomers having the formulae (Ia-Ic)

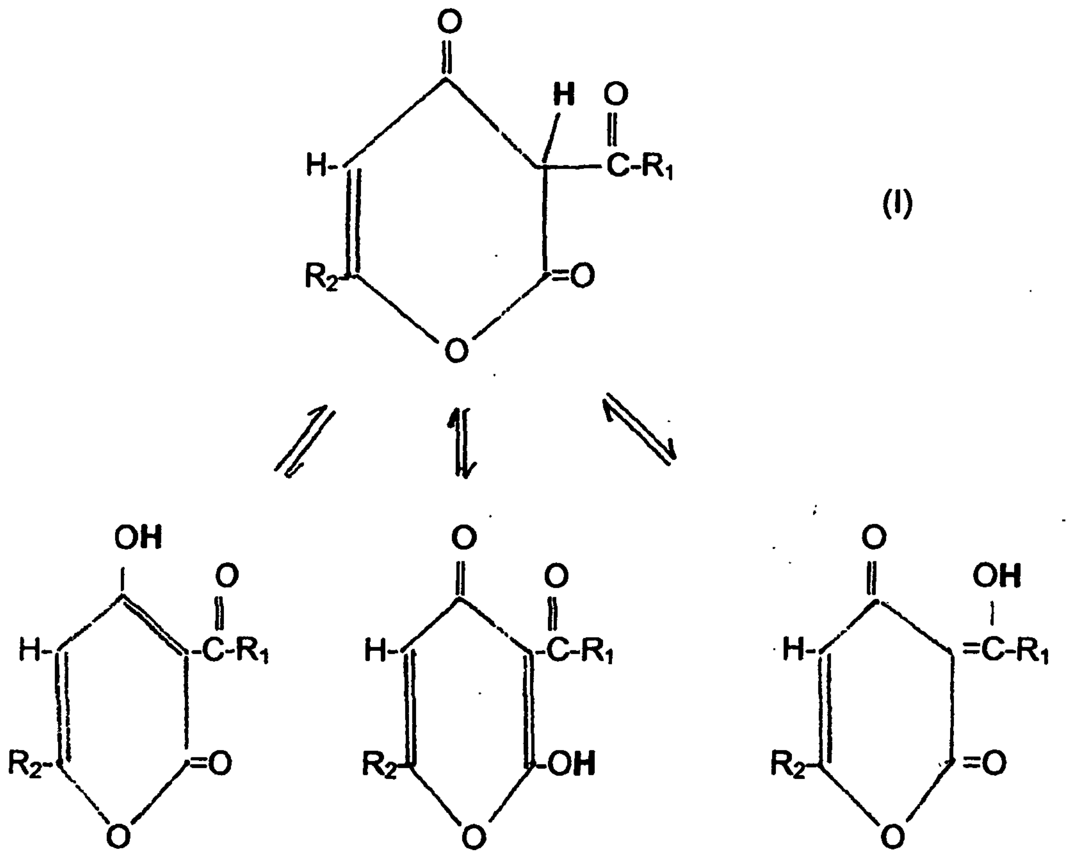

(I)

(Ia) (Ib) (Ic)

wherein
$R_1$ and $R_2$ are identical or different, where
$R_1$ is phenyl, $C_{1-4}$-alkylphenyl, especially benzyl, $C_{1-4}$-alkyl, $C_{1-4}$-alkenyl or $C_{1-4}$-alkinyl and
$R_2$ is COOH or COOR (wherein R is defined as $R_1$) or has the same meanings as $R_1$ and
wherein the OH-groups optionally may be partly or completely replaced by halide, especially Cl and/or Br radicals or by oxyglycoside, thioglycoside and/or aminoglycoside radicals,
and/or one or more salts, especially alkaline earth metal, alkaline metal and/or ammonium salts, thereof,
in an amount of from 0.01 to 0.5 wt.%, based on the total weight of the cosmetic omposition.

2. The cosmetic composition according to claim 1, **characterized in that** it contains, as an active ingredient, at least one enol-lactone having the formulae (I), (Ia), (Ib) and (Ic) as stated in claim 1 wherein $R_1$ and $R_2$ being identical or different are $CH_3$ or $C_2H_5$.

3. The cosmetic composition according to any of claims 1 to 2, **characterized in that** it contains, as an active ingredient, 3-acyl-3,4-dihydro-6-alkyl-2H-pyran-2,4-dion (ADAPD) having the formula (I) or (Ia, Ib, Ic), respectively, wherein $R_1$ and $R_2$ are $CH_3$ (methyl-ADAPD) or are $C_2H_5$ (ethyl-ADAPD) or a salt, especially a sodium salt, thereof.

4. The cosmetic composition according to any of claims 1 to 3, **characterized in that** it contains the active ingredient (s) having the formula (I) or (Ia, Ib, Ic), respectively, in an amount of from 0.15 to 0.5 wt.%, based on the total weight of the cosmetic composition.

5. The cosmetic composition according to any of claims 1 to 4, **characterized in that** it contains, as additional additive (s), aldohexonic acids, ketohexonic acids, tartaric acid, citric acid and/or ascorbic acid.

6. The cosmetic composition according to claim 5, **characterized in that** it contains the additional additives in an amount of from 0.01 to 5 wt.%, preferably in an amount of from 0.1 to 0.5 wt.%, based on the total weight of the cosmetic composition.

7. The cosmetic composition according to any of claims 1 to 6, **characterized in that** it contains, as further additive (s), S-containing amino acids and/or S-containing oligopeptides, especially glutathiones.

8. The cosmetic composition according to claim 7, **characterized in that** it contains the further additives in an amount of from 0.01 to 3.0 wt.%, preferably in an amount of from 0.1 to 0.5 wt.%, based on the total weight of the cosmetic composition.

9. The cosmetic composition according to any of claims 1 to 8, **characterized in that** it contains, as further ingredient (s), keratolytically active compounds, especially keratases and/or $\alpha$-hydroxy acids (AHA acids).

10. The cosmetic composition according to claim 9, **characterized in that** it contains, as further ingredients, rubiginic acid, hydracetic acid, comenic acid, chelidonic acid and/or meconic acid.

11. The cosmetic composition according to claim 9 or 10, **characterized in that** it contains the further ingredient(s) in an amount of from 0.01 to 5.0 wt.%, preferably in an amount of from 0.1 to 0.5 wt.%, based on the total weight of the cosmetic composition.

12. The cosmetic composition according to any of claims 1 to 11, **characterized in that** it contains furthermore sorbitol, urea, a succinate, ethanol, dexpanthenol as well as common cosmetic additives, auxiliary agents, vehicles etc.

13. The cosmetic composition according to any of claims 1 to 12, **characterized in that** it has the form of a lotion, a cream, an ointment, a solution, a gel or a spray.

14. A process for preparing the cosmetic composition according to claims 1 to 13,
**characterized in**
**that** one or more active ingredients having the formula (I) and/or tautomers and/or derivatives thereof are added to a solution or dispersion of the other ingredients of the cosmetic composition which solution or dispersion has been prepared in a manner known per se and adjusted to a pH between 6 and 7, especially between 6.3 and 6.7,
and **that** the obtained mixture is vented, homogenized, coldly stirred and optionally passed through an ointment preparation chair (an apparatus for preparing an ointment) in a manner known per se.

15. Use of the cosmetic composition according to claims 1 to 14, especially of the ADAPD ingredients having the general formulae (I, Ia, Ib, Ic), in particular of methyl-ADAPD and/or ethyl-ADAPD, contained therein, for preventing or reducing the formation or for removing brown spots from the skin of animals and human beings.

## Revendications

1. Composition cosmétique ayant un « effet blanchissant » et contenant comme substance active au moins un énol-lactone de la formule (I) générale indiquée ci-dessous et/ou un ou plusieurs de ses tautomères des formules (Ia-Ic)

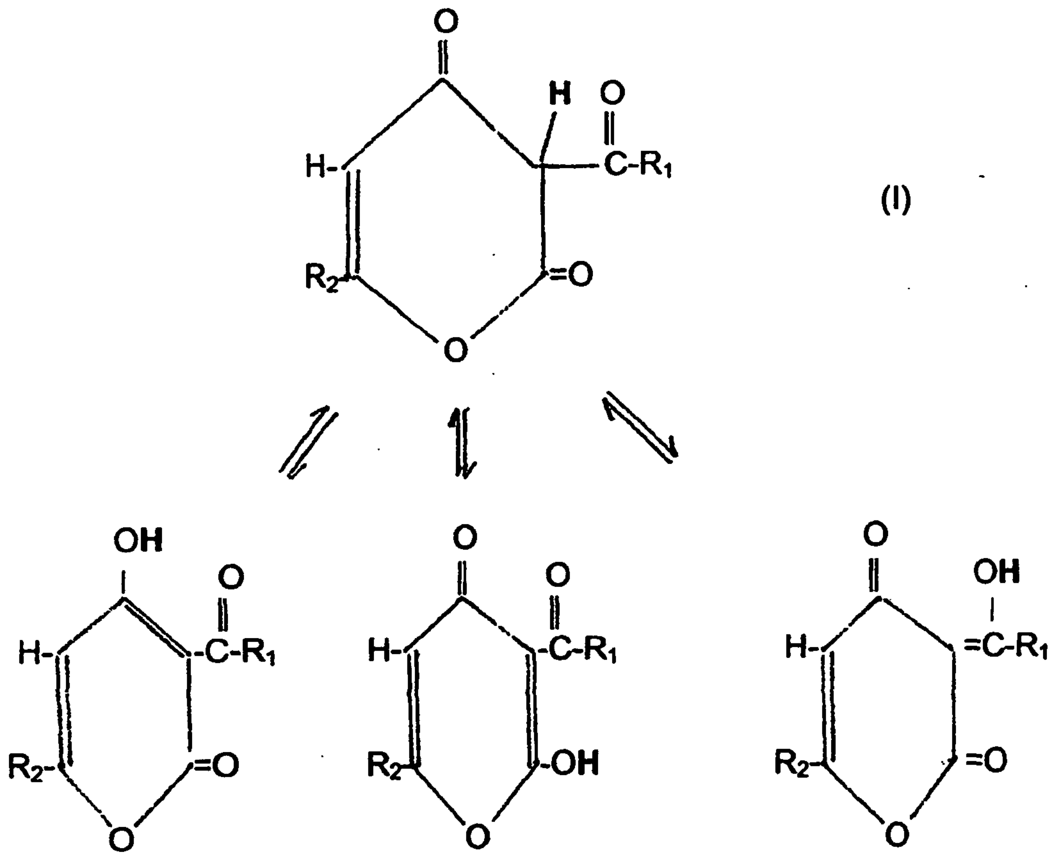

(I)

(Ia) (Ib) (Ic)

dans lesquelles
$R_1$ et $R_2$ sont identiques ou différents où
$R_1$ représente le phényle, le $C_{1-4}$-alkyle-phényle, en particulier le benzyle, le $C_{1-4}$-alkyle, le $C_{1-4}$-alcényle ou le $C_{1-4}$-alcynyle et
$R_2$ représente COOH ou COOR (où R a les significations indiquées pour $R_1$) ou les mêmes résidus que $R_1$ et dans lesquelles les groupes OH peuvent être remplacés, le cas échéant pour partie ou entièrement, par des résidus d'halogénure, en particulier Cl et/ou Br, ou bien par des résidus d'oxyglycoside, de thioglycoside et/ou d'aminoglycoside,
et/ou un ou plusieurs sel(s), en particulier sels alcalinoterreux, sels alcalins et/ou sels d'ammonium
en une quantité allant de 0,01 à 0,5 % en poids par rapport au poids total de la composition cosmétique.

2. Composition cosmétique selon la revendication 1, **caractérisée en ce qu'**elle contient comme substance active au moins un énol-lactone des formules (I), (Ia), (Ib) et (Ic) indiquées dans la revendication 1, dans lesquelles $R_1$ et $R_2$ sont identiques ou différents et représentent $CH_3$ ou $C_2H_5$.

3. Composition cosmétique selon l'une des revendications 1 à 2, **caractérisée en ce qu'**elle contient comme substance active l'acyle-3-dihydro-3,4-alkyle-6-pyrane-2H-dione-2,4 (ADAPD) de la formule (I) respectivement (Ia, Ib, Ic) dans lesquelles $R_1$ et $R_2$ représentent $CH_3$ (méthyle-ADAPD) ou $C_2H_5$ (éthyle-ADAPD) ou un sel du même, en particulier un sel de sodium.

4. Composition cosmétique selon l'une des revendications 1 à 3, **caractérisée en ce qu'**elle contient la(les) substance(s) active(s) de la formule (I) respectivement (Ia, Ib, Ic) en une quantité allant de 0,15 à 0,5 % en poids par rapport au poids total de la composition cosmétique.

5. Composition cosmétique suivant l'une des revendications 1 à 4, **caractérisée en ce qu'**elle contient en additif(s)

supplémentaire(s) des acides aldohexoniques, des acides kétohexoniques, de l'acide tartrique, de l'acide citrique et/ou de l'acide ascorbique.

6. Composition cosmétique selon la revendication 5, **caractérisée en ce qu'**elle contient les additifs supplémentaires en une quantité allant de 0,01 à 5 % en poids, de préférence en une quantité allant de 0,1 à 0,5 % en poids, par rapport au poids total de la composition cosmétique.

7. Composition cosmétique selon l'une des revendications 1 à 6, **caractérisée en ce qu'**elle contient en additif(s) supplémentaire(s) des acides aminés sulfurifères et/ou des oligopeptides sulfurifères, en particulier des glutathions.

8. Composition cosmétique selon la revendication 7, **caractérisée en ce qu'**elle contient les additifs supplémentaires en une quantité allant de 0,01 à 3,0 % en poids, de préférence en une quantité allant de 0,1 à 0,5 % en poids, par rapport au poids total de la composition cosmétique.

9. Composition cosmétique selon l'une des revendications 1 à 8, **caractérisée en ce qu'**elle contient en additif(s) supplémentaire(s) des composés à effet kératolytique, en particulier de la kératase et/ou des $\alpha$-hydroxy-acides (acides AHA).

10. Composition cosmétique selon la revendication 9, **caractérisée en ce qu'**elle contient comme substances actives supplémentaires de l'acide rubiginique, de l'acide hydracétique, de l'acide coménique, de l'acide chélidonique et/ou de l'acide méconique.

11. Composition cosmétique selon la revendication 9 ou 10, **caractérisée en ce qu'**elle contient la(les) substance(s) active(s) supplémentaire(s) en une quantité allant de 0,01 à 5,0 % en poids, de préférence en une quantité allant de 0,1 à 0,5 % en poids, par rapport au poids total de la composition cosmétique.

12. Composition cosmétique selon l'une des revendications 1 à 11, **caractérisée en ce qu'**elle contient, par ailleurs, du sorbitol, de l'urée, un succinate, de l'éthanol, du dexpanthénol ainsi que des additifs, excipients, véhicules etc. cosmétiques courants.

13. Composition cosmétique selon l'une des revendications 1 à 12, **caractérisée en ce qu'**elle est disponible sous forme de lotion, de crème, de pommade, de solution, de gel ou de spray.

14. Procédé pour préparer la composition cosmétique selon les revendications 1 à 13, **caractérisé en ce qu'**on ajoute une ou plusieurs substance(s) active(s) de la formule (1) et/ou des tautomères et/ou des dérivés de ceux-là à une solution ou dispersion, formée d'une manière connue en soi, des autres composants de la composition cosmétique après que la solution respectivement la dispersion a été amenée à une valeur de pH comprise entre 6 et 7, en particulier entre 6,3 et 6,7,
et **que** le mélange ainsi obtenu est alors, d'une manière courante, ventilé, homogénéisé, mélangé à froid et, le cas échéant, passé sur une chaise de pommade (un appareil de fabrication de pommades).

15. Utilisation de la composition cosmétique selon les revendications 1 à 14, en particulier des substances actives ADAPD mentionnées dedans ayant des formules générales (I, Ia, Ib, Ic), tout spécialement du méthyle-ADAPD et/ou du éthyle-ADAPD, afin d'empêcher la formation des tâches cutanées marron sur l'homme et l'animal, respectivement de les atténuer ou de les enlever.